# NEW EUROPEAN PATENT SPECIFICATION

(11) **EP 0 486 561 B2**
(45) Date of publication and mention of the opposition decision: **22.07.1998**
(45) Mention of the grant of the patent: 01.02.1995
(21) Application number: 90912165.9
(22) Date of filing: 16.08.1990
(51) Int. Cl.: A61K 7/16, A61K 7/18, A61K 31/19

(54) **BUCCAL COMPOSITION CONTAINING S(+) KETOPROFEN**
ORALES S(+) KETOPROFENHALTIGES MITTEL
COMPOSITION POUR LA BOUCHE CONTENANT DU S(+) KETOPROFENE

(30) Priority: 17.08.1989 US 395331
(43) Date of publication of application: 27.05.1992
(73) Proprietor: SEPRACOR, INC., Marlborough, MA 01752 (US)
(72) Inventor: WECHTER, William, J., Redlands, CA 92373 (US)
(74) Representative: Holdcroft, James Gerald, Dr.
(86) International application number: US9004623
(87) International publication number: WO9102512

(56) References cited:
- EP-A- 0 137 668
- WO-A-87/04618
- WO-A-88/03021
- WO-A-89/04658
- GB-A- 1 550 139
- US-A- 4 927 854
- J. Caldwell, A.J. Hutt and S. Fournel-Gigleux, Biochemical Pharmacology, 1988, pp. 105-114
- American Society for clinical Pharmacology and Therapeutics, february 1987, p. 162
- Nonsteroidal anti-inflammatory drugs, J. Lombardino (ed.), Wiley-Interscience, 1985, pp. 267-71, 308-309, 313-314, 400, 408-411
- J. Periodontol, vol. 50, n 9, 1979, Nyman S. et al, pp. 450-461
- Journal of periodontal research, vol. 16, 1981, Waite, I.M. et al, pp. 100-108
- J. Periodont. Res., vol. 23, 1988, Williams J.M.K. et al, pp. 381-385
- Clin. Pharmacol. Ther., 1987, Sunshine A. et al, p. 162
- The Journal of Biological Chemistry, vol. 260, n 23, 1985, Kulmacz R.J. et al, pp. 12572-12578
- J. Pharm. Pharmacol., vol. 35, 1983, Hutt A.J., pp. 693-704
- Drugs, vol. 30, 1985, Williams K. et al, pp. 333-354
- TIPS, vol. 9, n 3, 1985, Smith R.L. et al, pp. 75-77
- Clinical pharmakinetics, vol. 9, 1984, Hutt A.J. et al, pp. 371-373
- Pharm. Res., vol. 5, n 10, 1988, Berry B.W. et al, p. 192
- Clin. Pharm. Ther. vol. 43, 1988, Knadler M.P. et al, p. 135
- J. Pharmacology and experimental therapeutics, vol. 249, n 2, 1989, Knadler M.P. et al, pp. 378-385
- J. Medicinal Chemistry, vol. 18, n 1, 1975, Greig M.E. et al, pp. 112-116
- J. Pharmaceutical Sciences, vol. 77, n 8, 1988, Jamali F. et al, pp. 666-669
- R.M. Palmer and P.D. Floyd "A clinical guide to periodontology", 1996, pp. 7-9, 78-79
- R.A. Seymour et al, "Adverse drug reactions in dentistry", 1996, Oxford University Press, p. 96-100
- I.L. Sperling, the Lancet II, 535 (1969), pp. 535-537
- J. Guggenheimer et al, J. AM. Dent. Assoc. 90, 1975, pp. 632-634
- G.S.N. Kaziro, Aust. Dent. J., 25, pp. 333-334

## Description

### Background

Periodontal disease, which includes any abnormality, whether inflammatory or degenerative, of tissue around a tooth, is very common worldwide. For example, the World Health Organization has estimated that even if there were no new periodontal disease, it would take 45 years to treat those people who are affected. In addition, the Journal of Public Health Dentistry concluded in 1985 that "more than two out of three patients were affected by periodontal disease." Moos, W.F., Medical Marketing & Media, 52-54 (1985). Chronic gingivitis (i.e., inflammation of the gingiva or gums) and chronic destructive periodontitis (i.e., a disease of the connective tissue which attaches a tooth to the alveolar bone, which results in alveolar bone resorption, increasing mobility of the tooth and, ultimately, tooth loss) are two common types of periodontal disease.

Although chronic periodontal disease is so common and known to be caused ultimately by bacteria accumulated on the teeth and under the gingiva, progress in its prevention and treatment has been limited and therapy is still largely unsuccessful. Preventive techniques rely heavily on establishing and maintaining good oral hygiene and therapy of existing periodontal disease includes expensive and ongoing treatments, such as periodontal surgery, which, in many cases, must be carried out often and has not been clearly shown to be effective in arresting alveolar bone loss and preserving teeth. Alveolar bone loss or resorption, which occurs after tooth extraction, is also a serious dental problem which cannot, to date, be successfully treated. A more effective method of preventing or treating alveolar bone loss would be of great value, particularly because of the prevalence of its occurrence.

Compositions comprising racemate of ibuprofen or flurbiprofen have already been used for preventing or inhibiting alveolar bone resorption (EP-A-0 137 668) as well as for the treatment of gingivitis (WO 88/03021). The compositions according to EP-A-0 137 688 comprised non-anti-inflammatory quantities of active agent.

### Summary of the Invention

The present invention relates to the use of a composition comprising the purified S(+) enantiomer of Ketoprofen for the manufacture of a topical medicament having enhanced bioavailability for the treatment of periodontal bone loss to reduce bone resorption and inflammation and to promote bone regrowth. The present invention finds particular application in the case of alveolar bone loss associated with periodontal disease, bone loss associated with osteoporosis and fracture repair. The invention may find application in a method whereby S(+)ketoprofen, is administered by application to the buccal membranes to an individual, in whom bone loss is to be prevented, reduced or reversed, in sufficient quantities to produce a systemic effect (adequate blood levels of the drug). In the case in which an individual with periodontal disease requires treatment, the invention is also useful in reducing (decreasing or preventing) inflammation or gingivitis. The present invention may involve a composition which can be used to provide a means by which the S enantiomer of the nonsteroidal anti-inflammatory drug can be applied to make sufficient contact with an individual's buccal membranes to result in adequate blood levels of the drug to produce the desired effect. Typically, the composition will be a formulation, referred to as a toothpaste, but which can be any form (gel, powder, foam) or a mouthwash. The toothpaste is used as is any other toothpaste (typically, it is applied by brushing), as is the mouthwash, with which an individual gargles, rinses his or her mouth, etc.

Such a composition may include the S(+)enantiomer of the nonsteroidal anti-inflammatory drug, either in highly purified form (i.e., substantially free of its R(-) form) or in combination with a small quantity of the R(-) form. In addition, the composition includes a flavoring agent or agents and other components typically present in toothpastes (gels, powders, foams, etc.). Because the S(+) enantiomer, which is readily absorbed and is likely to have enhanced bioavailability, is used in the composition, a lower concentration is needed than would be the case if the typically-used racemic mixture were included in the composition. Typically, the nonsteroidal anti-inflammatory drug will be present in the composition in a concentration of approximately 0.1 to approximately 5.0% and preferably in a concentration of approximately 0.25% to approximately 1.0%, although the level can be altered as needed. In the case in which two or more such enantiomers are present, the total concentration falls within this range. The present composition and the method of using it in preventing or treating bone resorption and inflammation secondary to periodontitis and in promoting bone regrowth once it has occurred have several advantages over other formulations or methods of treating this condition. For example, because the active component of the composition is the highly purified S(+) enantiomer of the nonsteroidal anti-inflammatory drug, a relatively low concentration is needed and, thus, the problems of unpleasant or bitter taste and irritation to tissues of the mouth and/or other areas of the gastrointestinal tract associated with consumption of such drugs can be avoided. In addition, because the S(+) enantiomer is well absorbed, it is possible, using such formulations, to produce adequate blood levels (i.e., levels high enough to reduce bone resorption, such as that associated with periodontal disease), reduce inflammation associated with periodontal disease and/or promote regrowth of bone once loss has occurred.

### Detailed Description of the Invention

The present invention is based on the use for the manufacture of a topical medicament of the S(+) enantiomer of ketoprofen, in a composition for oral administration for the treatment and/or prevention of bone resorption.

The S(+) enantiomer of ketoprofen is administered by application to an individual's buccal membranes in order to reduce (decrease or prevent) inflammation and alveolar bone loss associated with periodontal disease and/or to promote bone regrowth associated with the disease. As used herein, the term periodontal disease refers to any disease which affects the periodontia and, typically includes periodontitis, gingivitis and/or periodontosis. The invention can also be used in a method wherein bone loss associated with other conditions is reduced, such as osteoporosis as well as to promote bone regrowth, once loss has occurred. It can also be used in conjunction with fracture repair. The S enantiomer is typically the dextrorotatory enantiomer and is designated S(+) using standard chemical notation. Further description of the present method and composition used therein will refer to the S(+) enantiomer, although it is not to be construed as limiting (i.e., the active enantiomer is what is intended).

In the case in which the S(+) enantiomer-containing composition is administered to treat or prevent alveolar bone loss and/or to promote alveolar bone regrowth associated with periodontal disease, the composition is a formulation, referred to for convenience as a toothpaste, although it may take other forms which can be applied to the gum area by brushing or other means of topical application, or is a mouthwash. The toothpaste may be, for example, a gel, powder, or a foam which is applied to the gums and then removed (e.g., by further or continued brushing with a toothbrush which does not contain the formulation or by rinsing with water). In general, the toothpaste formulation of the present invention contains from approximately 0.1% to 5.0% of the S(+) enantiomer and, preferably, from approximately 0.25% to approximately 1.0% of the S(+) enantiomer in highly purified form, although this concentration can be varied as needed in a particular instance. The toothpaste is applied in sufficient quantity (e.g., 1-2 grams toothpaste, twice daily) and for sufficient time to produce adequate blood levels (an adequate systemic level) to result in the desired effect. It also appears that this results in localized tissue levels which are of value in producing the desired effect.

The S(+) enantiomer of ketoprofen have been shown to be readily absorbed when taken orally. In general, because these drugs are themselves acids, the composition (toothpaste, mouthwash) should be acidic (e.g., pH of 5.0 to 6.5) to enhance the absorption of the drug(s). The selected S(+) enantiomer can be incorporated into an existing toothpaste formulation, simply by mixing, or can be included with other components as they are combined. An important consideration in terms of user acceptance and willingness to comply with a use regimen is inclusion in the formulation of a flavoring material (e.g., menthol, spearmint, peppermint) sufficiently strong to cover or reduce the flavor of the S(+) enantiomer, which is generally regarded as unpleasant because it is bitter, as well as to reduce the burning sensation it can cause. Because the S(+) enantiomer is used, however, masking or reducing the unpleasant flavor is not as difficult as would be the case if the racemic mixture were used because of the considerably smaller quantity of the S(+) enantiomer used (e.g., approximately one half that of the racemic mixture) and, thus, the lower intensity of the unpleasant flavor.

In the present method, the S(+) enantiomer-containing formulation is applied in sufficient quantity (e.g., generally 1-2 grams of a 0.25% to 1.0% toothpaste), twice a day. Thus, the amount of S(+) enantiomer that is applied twice daily can range from 1-50mg, but usually ranges from 2.5-20mg. Because the S(+) enantiomer of ketoprofen is used and can be incorporated into a formulation which is acceptable to an individual and convenient for self administration/home use, the present method and composition avoid an important limitation of previously-described methods, in which using racemic mixtures (S(+), R(-)) of either or both compound(s) were used and must be administered in tablet or other form which did not remain in the mouth for any length of time. A particular advantage of the present method and formulation is that it can easily be administered on an on-going basis and user compliance will be high.

The ability of a particular (selected) formulation to have the desired effect (i.e., reduce inflammation, reduce bone loss, promote regrowth) can be assessed using standard techniques. For example, its effect on inflammation is determined by observation, to determine whether the redness and/or puffiness or edema characteristic of inflammation has decreased. Bone loss reduction can be assessed using the method of Jeffcoat and co-workers. Jeffcoat, M.K. et al., J. Periodont. Res., 23:381-385 (1988). As described by Jeffcoat et al., the bone-seeking radiopharmaceutical 99m-Tc-MDP is used to assess the effect of a nonsteroidal anti-inflammatory drug (in the instant case, S(+) ketoprofen) in reducing periodontal disease activity. As applied to assessment of the effects of the S(+) enantiomers, alveolar bone height is determined using standardized radiography and alveolar bone metabolism is assessed using 99m-Tc uptake prior to administration of the selected S(+) enantiomer and [2] months after administration begins. A reduction in radiopharmaceutical (99m-Tc) uptake, after S(+) enantiomer administration, in the alveolar bone of teeth shown initially (prior to administration) to be undergoing active bone loss is interpreted as an indication that the S(+) enantiomer used has a beneficial effect (i.e., reduces alveolar bone loss in individuals with periodontal disease). Bone regrowth can be assessed using a standard method, such as digital subtractive radiography (see Jeffcoat et al.).

S(+) ketoprofen may be administered orally to reduce bone resorption and/or promote bone growth associated with conditions or diseases other than periodontal disease, such as osteoporosis and fracture repair. Here, the selected S(+) enantiomer is applied in a similar manner, resulting in the desired systemic effect (i.e., blood levels appropriate for affecting bone metabolism).

The S enantiomer used in the method and composition of the present invention can be produced by any appropriate method. For example, it can be produced by the method described in WO 89/09765. This patent teaches a combination of organic synthesis and enzymatic treatment to produce the desired enantiomer of drugs such as flurbiprofen or ketoprofen.

The following Example details the protocol for assessing the bioavailability of toothpastes containing various levels of S(+) flurbiprofen. The bioavailability of S(+) ketoprofen can also be determined using this protocol.

### EXAMPLE Evaluation of Bioavailability of S(+) Flurbiprofen in Toothpaste - not according to the invention

The purpose of this study is to show how well subjects tolerate three strengths of S(+) flurbiprofen toothpaste (1%, 0.5% and 0.25%), as well as the relationship between dose and blood level of the active isomer in comparison with an identical formulation of racemic flurbiprofen (1%). A toothpaste containing one of the three concentrations of S(+) flurbiprofen or 1% racemic flurbiprofen is used in the study.

The drug is an acid and, therefore, the formulation is adjusted to be on the acid side, in order to improve buccal absorption. Any standard formulation of appropriate pH can be used. The formulation can also contain menthol or wintergreen in order to mask the slight bitter taste of the drug. Standard formulation techniques are used.

Subjects are selected as follows: Seven males, all of whom are in good health (age 21) are recruited in order to allow for one dropout during the course of the study. Individuals with allergies to aspirin or nonsteroidal anti-inflammatory drugs; asthma; a history of valvular heart disease; or a history of ulcer disease or chronic dyspepsia are excluded.

A 7 ml. sample of blood is drawn from each subject and serves as the time zero reading (prior to any brushing) for serum flurbiprofen. Over a period of four days, following an initial oral examination, six subjects brush their teeth twice daily with one of the four toothpaste formulations. The subjects keep a daily diary recording the times of their morning and evening brushings and any observed effects.

After their morning brushing on the fourth day, a single 7 ml sample of blood is drawn from each subject immediately after brushing, then again at .25 hours, 1 hour, 8 hours, 18 hours and 24 hours. Blood samples are centrifuged and the serum frozen at approximately -10° C prior to delivery to a designated analytical laboratory.

Serum samples are analyzed to determine the concentration of flurbiprofen stereoisomers.

This same process is repeated after three days through three additional cycles until all subjects have received all four treatments. The dosage strengths are randomized throughout the four experimental cycles.

At completion of the four treatments the subjects are again examined in order to evaluate effects on the subjects' oral cavities (i.e., as described previously, effects on inflammation, side effects).

## Claims

1. Use of a composition comprising the purified S(+) enantiomer of ketoprofen for the manufacture of a topical medicament having enhanced bioavailability for the treatment of periodontal bone loss, to reduce bone resorption and inflammation and to promote bone regrowth.

2. Use according to claim 1, wherein the composition includes from 0.1% to 5.0% of the purified S(+) enantiomer.

3. Use according to claim 1 or claim 2 wherein the medicament is in the form of a toothpaste or mouthwash.

## Patentansprüche

1. Verwendung einer Zusammensetzung, die das gereinigte S(+)-Enantiomere von Ketoprofen enthält oder daraus besteht, zur Herstellung eines topischen Medikaments mit erhöhter biologischer Verfügbarkeit zur Behandlung von periodontalem Knochenverlust, um Knochenresorption und Entzündung herabzusetzen und neues Knochenwachstum zu fördern.

2. Verwendung nach Anspruch 1, wobei die Zusammensetzung 0,1 % bis 5,0 % des gereinigten S(+)-Enantiomeren aufweist.

3. Verwendung nach Anspruch 1 oder 2, wobei das Medikament in Form einer Zahncreme oder eines Mundwasser ist.

## Revendications

1. Utilisation d'une composition comprenant l'énantiomère purifié S(+) du kétoprofène pour la préparation d'un médicament topique présentant une biodisponibilité accrue pour le traitement d'une perte osseuse parodontale afin de réduire la résorption osseuse et l'inflammation et favoriser la repousse osseuse.

2. Utilisation selon la revendication 1, selon laquelle la composition comprend de 0,1 à 5,0% de l'énantiomère purifié S(+).

3. Utilisation selon la revendication 1 ou la revendication 2, selon laquelle le médicament est sous la forme d'une pâte dentrifice ou d'un bain de bouche.
